# FASCICULE DE BREVET EUROPEEN

(11) **EP 1 185 684 B1**
(45) Date de publication et mention de la délivrance du brevet: **16.02.2005**
(21) Numéro de dépôt: 00938893.5
(22) Date de dépôt: 06.06.2000
(51) Int. Cl.: C12Q 1/00

(54) **BIOCAPTEUR ELECTROCHIMIQUE ET PASTILLE POUR UN TEL BIOCAPTEUR**
ELEKTROCHEMISCHER BIOSENSOR UND EINSATZELEMENT DAFÜR
ELECTROCHEMICAL BIOSENSOR AND CHIP FOR SUCH A BIOSENSOR

(30) Priorité: 10.06.1999 FR 9907337
(43) Date de publication de la demande: 13.03.2002
(73) Titulaire: Grasa, Jean-Pierre, 30760 Saint Julien de Peyrolas (FR)
(72) Inventeur: Grasa, Jean-Pierre, 30760 Saint Julien de Peyrolas (FR)
(74) Mandataire: Cabinet BARRE LAFORGUE & associés
(86) Numéro de dépôt international: PCT/FR2000/001546
(87) Numéro de publication internationale: WO 2000/077240

(56) Documents cités:
- EP-A- 0 546 536
- EP-A- 0 600 607
- WO-A-90/00738
- US-A- 5 208 147
- US-A- 5 437 999
- US-A- 5 711 862

## Description

L'invention concerne un biocapteur électrochimique de mesure de la concentration d'un composé dans une dose échantillon d'une solution liquide.

Le principe et l'intérêt des biocapteurs sont connus depuis longtemps (cf. par exemple la publication "BIOCAPTEURS : REVE OU REALITE INDUSTRIELLE ?", Maurice Comtat et Alain Bergel, BIOFUTUR 171, octobre 1997 p 33). Néanmoins, leurs applications pratiques restent limitées compte tenu de leur difficulté de mise en oeuvre.

En particulier le problème se pose du dosage, de la mise en place et du renouvellement de la composition biochimique réactive (notamment formée d'une enzyme telle que la glucose oxydase) spécifique du composé dont on veut mesurer la concentration.

Dans les appareils connus tels que ceux commercialisés par INCELTECH FRANCE (Toulouse, France) sous la dénomination MICROZYM-L ®, le biocapteur comprend une colonne dont l'âme forme une première électrode débouchant, à l'extrémité de la colonne dans une chambre de réactif creuse dans laquelle une quantité de solution réactive enzymatique est versée par le manipulateur. La chambre est ensuite refermée par une membrane semi-perméable coiffant l'extrémité de la colonne et maintenue pas un joint torique.

Une deuxième électrode entoure la colonne à distance du joint torique. La composition liquide est versée sur la membrane, l'extrémité de la colonne étant placée vers le haut. Le contact électrique est établi entre les deux électrodes via la solution réactive et la solution liquide échantillon qui s'écoule entre la membrane et la deuxième électrode.

Ces appareils nécessitent un grand volume de solution liquide à doser. Certains dispositifs ou appareils connus présentent une cellule de réaction comprenant un puits ou cuve de réception d'une dose de solution liquide. Dans ce cas, les puits doivent être changés à chaque dosage, ce qui est coûteux et implique des manipulations longues.

En outre, dans ces appareils, le changement de la composition réactive est long (plus de 10 minutes de manipulations) et délicat. De surcroît, les manipulations requises, relativement précises, sont possibles en laboratoire mais ne peuvent pas être envisagées dans un environnement hors laboratoires (industriel, agricole ....).

Egalement, ces appareils ne permettent pas la mesure de la concentration de différents composés, sauf à être équipés de plusieurs biocapteurs, un pour chaque composé à mesurer. Mais, dans tous les cas, la mise en oeuvre d'un biocapteur pour chaque composé est longue et délicate.

Une autre conséquence de cette difficulté de mise en place est le fait que la qualité de la manipulation peut influencer la reproductibilité et la fiabilité des mesures effectuées.

Une des solutions envisagées pour pallier ces problèmes consiste à immobiliser la composition réactive enzymatique sur une électrode ou sur la membrane semi-perméable. Néanmoins, les techniques d'immobilisation sont très difficiles à mettre en oeuvre. Parmi celles-ci, on peut citer : l'adsorption de l'enzyme sur un support de type colladion, collagène, cellulose, carbone, gel de silice .... ; l'inclusion de l'enzyme dans un gel ou une pâte carbonée ou une encre carbonée ; la fixation par liaison covalente de l'enzyme sur un support préalablement activé.

Toutes les solutions envisagées s'avèrent être complexes et coûteuses à la fabrication et à l'utilisation.

Par ailleurs, il est à noter que le coût important des compositions réactives (classiquement de l'ordre de 80 FF pour une dose de 4 µl de glucose oxydase) impose de pouvoir conserver la même composition réactive tant qu'elle n'est pas dégradée. Or, les compositions biochimiques réactives -notamment enzymatiques- sont relativement fragiles et instables.

L'invention vise donc à pallier l'ensemble de ces inconvénients en proposant un biocapteur électrochimique dans lequel la mesure peut être faite avec un volume faible de solution liquide, notamment une seule goutte, et dont l'utilisation est simple, rapide et peu coûteuse.

L'invention vise en particulier à proposer un biocapteur exempt de cellule de réaction en forme de puits ou cuve de réception de la solution liquide. L'invention vise aussi à proposer un biocapteur permettant d'effectuer des mesures successives rapidement, à moindre coût, en minimisant les temps morts entre deux mesures et les coûts unitaires de chaque mesure.

Par ailleurs, l'invention vise aussi à proposer un biocapteur dans lequel la mise en place de la composition réactive est extrêmement simple et rapide.

L'invention vise aussi à proposer un biocapteur permettant un changement rapide de composition réactive, par exemple pour la mesure de la concentration de differents composés successivement.

L'invention vise aussi à proposer un biocapteur fournissant des résultats fiables, y compris dans des environnements hors laboratoires (industrie, agriculture ...).

L'invention vise de surcroit à proposer un tel biocapteur qui soit de coût reduit tant à la fabrication qu'à l'utilisation.

L'invention vise ainsi à proposer un biocapteur permettant de satisfaire de nombreuses applications dans lesquelles les appareils connus ne peuvent pas être exploités en raison de la nature ou du nombre des composés dont la concentration peut être mesurée et ou du défaut de fiabilité des résultats et/ou des problèmes d'utilisation et de mise en oeuvre et/ou d'un coût trop élevé de ces appareils connus.

Pour ce faire, l'invention concerne un biocapteur électrochimique de mesure de la concentration d'un composé dans une solution liquide, comprenant :
une chambre de réactif adaptée pour renfermer une quantité de composition biochimique, dite composition réactive, et présentant une face externe libre, dite face de contact, apte à être placée au contact de la solution liquide qui est ainsi elle-même en contact avec la composition réactive,
une première et une deuxième électrodes adaptées pour être électriquement reliées à la solution liquide et permettre une mesure électrique entre elles,
caractérisé en ce qu'il comprend, en regard de la face de contact, un organe de retenue, la forme de cet organe de retenue et la distance entre la face de contact et l'organe de retenue étant adaptées pour qu'une goutte de solution liquide placée entre la face de contact et cet organe de retenue soit retenue et maintenue entre eux par capillarité. La solution liquide à analyser étant formée d'une goutte retenue par capillarité, on peut réaliser des dosages sur des solutions disponibles en faible volume, on réalise une économie sur la quantité de solution liquide nécessaire à la mesure, et les manipulations de mise en place et de nettoyage sont extrêmement simples et rapides. Par exemple, le nettoyage peut être réalisé simplement manuellement à l'aide d'un absorbant ou à l'air comprimé.

Avantageusement et selon l'invention, le biocapteur est caractérisé en ce que la première électrode est électriquement reliée à la chambre de réactif, à l'opposé de la face de contact, et en ce que la deuxième électrode est adaptée pour être électriquement reliée avec la goutte de solution liquide du côté de la face de contact. La deuxième électrode peut être adaptée pour être directement au contact de la goutte de solution liquide, ou, au contraire, être électriquement reliée à la goutte de solution liquide par au moins un élément intermédiaire électriquement conducteur (notamment un film ou canal de composition liquide conductrice et/ou la chambre de réactif elle-même).

Dans une variante selon l'invention, l'organe de retenue est formé de la deuxième électrode directement en contact avec la goutte de solution liquide, qui est alors interposée entre cette deuxième électrode et la face de contact de la chambre de réactif.

Dans une autre variante selon l'invention, le biocapteur est caractérisé en ce que l'organe de retenue est un organe spécifique distinct d'une électrode, et en ce que les deux électrodes sont électriquement reliées (directement ou indirectement) à la chambre de réactif.

De la sorte, une liaison électrique reste en permanence instaurée entre les deux électrodes via la chambre de réactif, évitant ainsi tout phénomène d'accumulation de charges (effets capacitif entre électrodes), et donc l'apparition d'un pic de décharge à la mise en place d'une goutte de solution liquide à analyser, nuisant à l'interprétation du signal.

Quoiqu'il en soit, avantageusement et selon l'invention, l'organe de retenue présente une face en regard de la face de contact. Ainsi, la goutte est retenue entre deux faces. En variante, il est possible de prévoir que l'organe de retenue se présente en forme de pointe en regard de la face de contact.

Par ailleurs, avantageusement et selon l'invention, l'ensemble formé de l'organe de retenue et de la face de contact présente une face, orientée vers le haut, dite face de réception de la goutte de solution liquide. Il est à noter néanmoins que la goutte étant retenue par capillarité, il est aussi possible d'envisager en variante que les faces ou éléments (organe de retenue et face de contact) entre lesquels elle est placée prennent toute position par rapport à la verticale, et notamment soient horizontalement en regard de l'autre.

Avantageusement et selon l'invention, ladite face de réception présente un angle d'inclinaison par rapport à l'horizontale, qui est supérieur à 0° et inférieur à 90° -notamment de l'ordre de 40°-. Cette inclinaison est adaptée pour permettre l'écoulement par gravité du surplus de solution liquide ou de la goutte chassée après la mesure.

Dans une première variante, la face de réception est la face de l'organe de retenue. Dans une autre variante préférentielle, la face de réception est la face de contact de la chambre de réactif.

Avantageusement et selon l'invention, la distance entre l'organe de retenue et la face de contact est inférieure à 8 mm. Quoiqu'il en soit, on sait déterminer la distance entre deux organes permettant l'insertion et le maintien par capillarité entre eux d'une goutte de solution à analyser. Cette distance est liée en particulier aux valeurs relatives des tensions de surface des organes (organe de retenue et face de contact) et de la solution liquide.

Par ailleurs, l'invention concerne aussi un biocapteur électrochimique de mesure de la concentration d'un composé dans une dose d'une solution liquide, ce biocapteur comprenant :
- une première électrode,
- une chambre de réactif adaptée pour renfermer une quantité de composition biochimique liquide, dite composition réactive, et la placer au contact de la première électrode, et comportant une membrane semi-perméable refermant la chambre de réactif de façon à y retenir la composition réactive, cette membrane semi-perméable présentant une face externe libre, formant ladite face de réception, apte à être placée au contact de la solution liquide séparée de la solution réactive par la membrane semi-perméable,
- une deuxième électrode disposée à distance de la membrane semi-perméable de façon à venir en contact avec la solution liquide placée contre la membrane semi-perméable,
caractérisé en ce qu'il comporte deux pièces distinctes :
- une pastille comprenant la première électrode, la chambre de réactif renfermant la composition réactive et la membrane semi-perméable,
- un bâti comprenant des moyens de réception d'une pastille, des moyens de connexion électrique, avec un circuit électrique extérieur, de la première électrode d'une pastille en place dans les moyens de réception, ce bâti portant la deuxième électrode à distance de la membrane semi-perméable d'une pastille en place dans les moyens de réception, et comprenant des moyens de liaison électrique de la deuxième électrode avec le circuit électrique extérieur.

La chambre de réactif préalablement dosée en composition réactive et incorporant la première électrode étant réalisée à l'avance et formée d'une pièce distincte du bâti qui est désignée dans tout le texte de façon générale par le terme "pastille", les manipulations et la mise en oeuvre du biocapteur sont grandement facilitées et rapides. Le changement de composition réactive est quasiment instantané.

Avantageusement et selon l'invention, la face de réception d'une pastille en place dans les moyens de réception est orientée vers le haut et présente un angle d'inclinaison non nul par rapport à l'horizontale. Cette inclinaison est adaptée pour permettre l'écoulement par gravité du surplus de solution liquide vers le bas, sans que celle-ci ne vienne directement au contact de la première électrode ou de ses moyens de connexion électrique disposés sous la pastille. Avantageusement et selon l'invention, ledit angle d'inclinaison est compris entre 0° et 90° - notamment de l'ordre de 40° -. Cette inclinaison peut être obtenue par une inclinaison d'une face du bâti recevant la pastille et/ou par une forme spécifique donnée à la pastille (par exemple en forme de coin).

Avantageusement et selon l'invention, la deuxième électrode comprend une extrémité libre s'étendant à distance et en regard de ladite face de réception de la membrane semi-perméable d'une pastille, et cette extrémité libre présente une face inclinée s'étendant au moins sensiblement parallèlement à la face de réception inclinée. Avantageusement et selon l'invention, la deuxième électrode présente une extrémité libre s'étendant à une distance de ladite face de réception de la membrane semi-perméable de la pastille, qui est adaptée pour retenir la goutte de solution liquide par capillarité -notamment inférieure à 8 mm-.

En outre, avantageusement et selon l'invention, le biocapteur est caractérisé en ce que la deuxième électrode s'étend au-dessus de la face de réception, et en saillie vers le bas par rapport à une face du bâti orientée vers le bas, de sorte que cette deuxième électrode présente au moins une face libre s'étendant vers le bas et orientée vers l'amont par rapport à l'inclinaison de la face de réception de la pastille, et en ce qu'il comprend un puits d'alimentation en solution liquide débouchant immédiatement à l'amont et en regard de la face libre de la deuxième électrode, de sorte que la solution liquide est alimentée et déposée sur cette face libre pour s'écouler vers le bas le long de la deuxième électrode jusqu'à venir dans l'interstice séparant la deuxième électrode et la face de réception inclinée.

Avantageusement et selon l'invention, le biocapteur est aussi caractérisé en ce que les moyens de réception d'une pastille comprennent un pan incliné orienté vers le haut (de façon à supporter une pastille placée sur ce pan incliné), et des moyens formant une butée de réception d'une pastille en position basse extrême sur le pan incliné, en ce que lesdits moyens de connexion électrique comportent un plot de contact électrique débouchant du pan incliné et adapté pour venir en liaison électrique avec une portion conductrice inférieure de la pastille en place et en butée sur le pan incliné, cette portion conductrice étant elle-même en liaison électrique avec la première électrode, et en ce que le bâti comprend un étrier portant la deuxième électrode au-dessus, en regard et à distance de ladite face de réception de la membrane semi-perméable d'une pastille en place et en butée sur le pan incliné. Avantageusement et selon l'invention, le biocapteur comprend des moyens pour plaquer la pastille contre le pan incliné. Avantageusement et selon l'invention, le bâti comprend un orifice de récupération de la solution liquide disposé de façon à pouvoir récupérer la solution liquide s'écoulant de la face de réception - notamment ménagé en partie inférieure du pan incliné - après la mesure et communiquant avec une extrémité inférieure du bâti. Avantageusement et selon l'invention, le biocapteur comprend des moyens de montage du bâti sur un récipient de récupération de la solution liquide.

L'invention vise aussi à proposer une pastille pour un biocapteur selon l'invention. A ce titre, l'invention vise à proposer un conditionnement fiable, de faible coût, facile à manipuler, pour une dose de composition biochimique liquide réactive - notamment une solution enzymatique - destinée à un biocapteur.

Pour ce faire, l'invention s'étend à une pastille de biocapteur électrochimique de mesure de la concentration d'un composé dans une solution liquide, caractérisée en ce qu'elle comprend :
- une chambre de réactif renfermant une quantité de composition biochimique liquide, dite composition réactive, et comportant une membrane semi-perméable refermant la chambre de réactif de façon à y retenir la composition réactive, cette membrane semi-perméable présentant une face externe libre, dite face de réception, apte à recevoir une goutte de solution liquide séparée de la solution réactive par la membrane semi-perméable,
- une électrode, dite première électrode, placée au contact électrique de la composition réactive contenue dans la chambre de réactif, et des moyens de connexion électrique de cette première électrode avec un circuit électrique extérieur à la pastille.

L'invention concerne aussi une pastille adaptée pour être utilisée avec un biocapteur selon l'invention. La pastille selon l'invention est simultanément un conditionnement consommable de composition réactive et un support pour la première électrode, qui peut être aussi bien une anode qu'une cathode, la deuxième électrode étant alors une cathode ou, respectivement, une anode.

Avantageusement et selon l'invention, la première électrode présente une extrémité débouchant dans la chambre de réactif à l'opposé d'une portion de la membrane semi-perméable formant ladite face de réception. Avantageusement et selon l'invention, la première électrode forme un fond de la chambre de réactif refermée, à l'opposé de ce fond, par la membrane semi-perméable. Avantageusement et selon l'invention, la chambre de réactif est délimitée par le fond formé par la première électrode et par la membrane semi-perméable s'étendant à partir du fond et au-dessus du fond. Avantageusement et selon l'invention, la pastille présente une gorge en creux autour du fond formé par la première électrode, cette gorge étant adaptée pour recevoir un joint périphérique bloquant la membrane semi-perméable autour et au-dessus de ce fond. Avantageusement et selon l'invention, la première électrode s'étend de façon à présenter une portion débouchant à l'extérieur de la pastille pour former des moyens de connexion électrique avec un circuit électrique extérieur. Avantageusement et selon l'invention, la pastille comprend un corps de matière synthétique électriquement isolante en forme générale de plaquette, et la première électrode traverse l'épaisseur de ce corps. En variante, la première électrode peut être reliée à un fil conducteur débouchant lui-même à l'extérieur de la pastille et adapté pour pouvoir être connecté au circuit électrique extérieur.

Avantageusement et selon l'invention, la pastille est en forme générale de disque. Une pastille selon l'invention présente avantageusement une épaisseur comprise entre 2 mm et 10 mm - notamment de l'ordre de 4 mm - et une dimension en longueur (ou diamètre) comprise entre 5 mm et 50 mm - notamment de l'ordre de 20 mm -. Avantageusement et selon l'invention, la première électrode présente une dimension radiale moyenne comprise entre 1 mm et 10 mm - notamment de l'ordre de 4 mm -.

Plus particulièrement et avantageusement, dans un biocapteur et une pastille selon l'invention, la composition réactive est une solution aqueuse enzymatique, et les électrodes sont du type à détection ampèromètrique (anode et cathode). Lorsque la composition réactive est une solution enzymatique incorporant une enzyme déshydrogènase avec un couple associé au couple Fe⁺⁺/Fe⁺⁺, la première électrode est une anode. L'invention s'étend néanmoins aussi à toute autre composition biochimique réactive, liquide ou non, compatible avec un biocapteur de type électrochimique à deux électrodes.

L'invention concerne aussi un biocapteur et une pastille caractérisés en combinaison par tout ou partie des caractéristiques mentionnées ci-dessus ou ci-après.

L'invention permet une manipulation aisée de la solution liquide à doser et du conditionnement (pastille) de la composition réactive et facilite grandement la mise en oeuvre et le renouvellement des doses de solution liquide à doser et de composition réactive, qui sont des opérations instantanées. De surcroît, la composition réactive est protégée de l'environnement extérieur et peut être conservée, stockée et réutilisée selon le composé dont la concentration est mesurée. L'invention peut aussi être mise en oeuvre et utilisée dans des environnements quelconques sans precautions particulières (industrie, exploitation agricole, terrain de sport ou en exterieur ...) de façon simple et permet d'obtenir des résultats fiables. Elle est en outre tres simple et peu coûteuse à fabriquer.

D'autres buts, caractéristiques et avantages de l'invention apparaitront à la lecture de la description suivante qui se réfère aux figures annexées dans lesquelles :
- la figure 1 est une vue schématique en perspective d'un biocapteur selon un premier mode de réalisation préférentiel l'invention,
- la figure 2 est une vue schématique en section par un plan vertical axial median du biocapteur de la figure 1.
- la figure 3 est une vue schématique de gauche du biocapteur de la figure 2.
- la figure 4 est une vue schématique en section selon la ligne IV-IV de la figure 2.
- la figure 5 est une vue schématique en perspective d'une pastille selon un premier mode de réalisation de l'invention,
- les figures 6a, 6b, 6c illustrent schématiquement en section par un plan vertical axial médian trois étapes successives de la fabrication de la pastille de la figure 5.
- la figure 7 est une vue schématique en section par un plan vertical axial médian d'une pastille selon un deuxième mode de réalisation de l'invention,
- la figure 8 est une vue schématique en section éclatée par un plan vertical axial médian illustrant les éléments constitutifs d'une pastille selon le deuxième mode de réalisation de l'invention.
- les figures 9, 10 et 11 sont des schémas partiels en section par un plan vertical axial médian illustrant trois autres modes de réalisation d'un biocapteur selon l'invention.

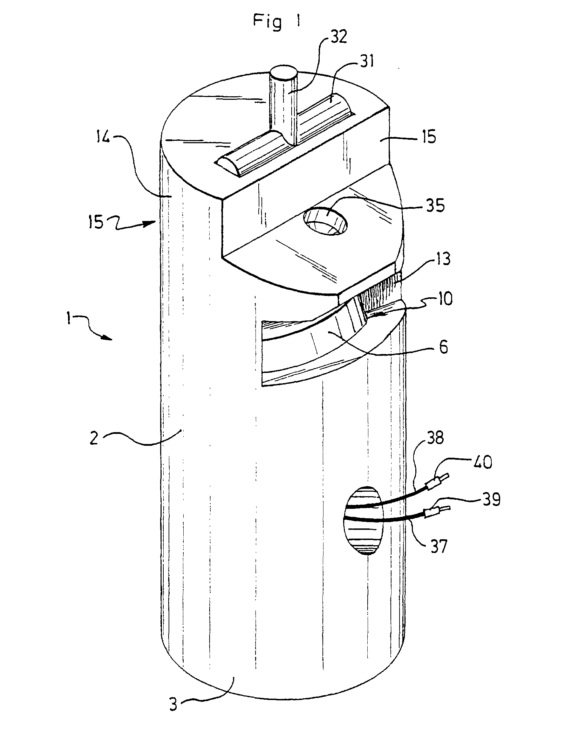

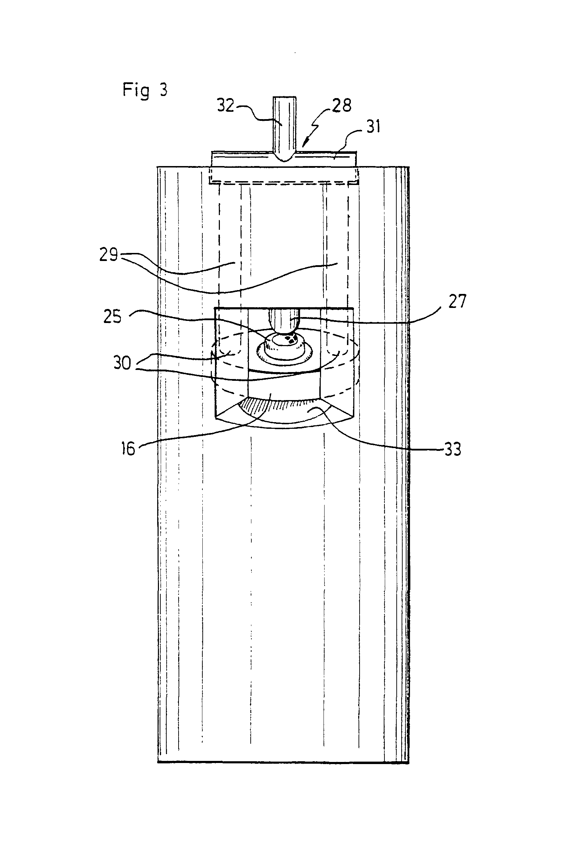

Le biocapteur 1 électrochimique selon l'invention représenté sur les figures 1 à 4, comprend un bâti 2 en matière synthétique électriquement isolante de forme générale globalement cylindrique de révolution à axe vertical, destiné à être monté par son extrémité inférieure 3 taraudée sur une extrémité supérieure 4 filetée d'un récipient tel qu'une bouteille destinée à recueillir des doses échantillons de solution liquide après analyse dans le biocapteur 1.

Le biocapteur 1 comprend une zone d'analyse 5 dans laquelle les doses de solution liquide à analyser peuvent être introduites. La zone d'analyse 5 incorpore les différents dispositifs permettant de réaliser les mesures de concentration dans la solution liquide et définit un compartiment de réception d'une pastille 6 formée d'une pièce distincte du bâti 2, et amovible par rapport au bâti 2.

Cette pastille 6 comprend une première électrode 7, une chambre de réactif 8 refermant une quantité prédéterminée de composition biochimique liquide, dite composition réactive, telle qu'une solution enzymatique, cette chambre de réactif 8 étant refermée par une membrane semi-perméable 9 de sorte que la composition réactive est retenue à l'intérieur de la chambre de réactif 8 et reste au contact de la première électrode 7.

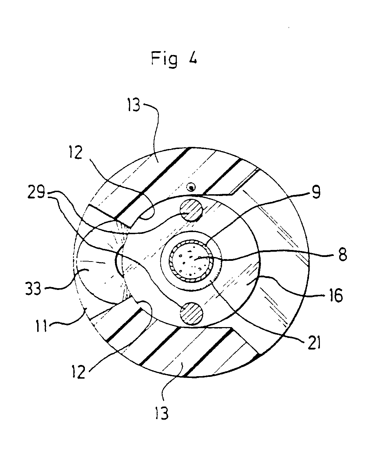

Le compartiment de réception de la pastille 6 comprend une fente d'introduction 10 latérale ménagée dans le bâti 2, un pan incliné 11 orienté vers le haut et s'étendant à partir de la fente 10 radialement vers le bas selon un angle d'inclinaison non nul qui est supérieur à 0° et inférieur ou égal à 90° - notamment de l'ordre de 40° - par rapport à l'horizontale et, à l'opposé de la fente 10 d'introduction, des moyens 12 formant une butée de réception de la pastille 6 en position basse extrême sur le pan incliné 11. Dans le mode de réalisation représenté, ces moyens 12 formant une butée sont constitués de deux montants latéraux 13 prolongeant le pan incliné 11 vers le haut de chaque côté et formant, avec une tête d'extrémité supérieure 14 du bâti 2, un étrier 15 qui vient coiffer le pan incliné 11 refermant le compartiment de réception de la pastille 6 et la zone d'analyse 5. Comme on le voit figure 4, les deux montants latéraux 13 présentent une forme adaptée pour recevoir entre eux une pastille 6 placée sur le pan incliné 11 tout en la bloquant en butée en position extrême inférieure sur le pan incliné 11.

La pastille 6 comprend un corps 16 en matière synthétique isolante de l'électricité, en forme générale de plaquette et de tronçon de cylindre de révolution de faible épaisseur, c'est-à-dire en forme générale de disque. Ce corps 16 est traversé dans son épaisseur axialement par la première électrode 7 qui est formée d'un matériau électriquement conducteur mais résistant à l'électrolyse. L'extrémité inférieure 17 de la première électrode 7 débouche à l'extérieur de la face inférieure du corps 16 de la pastille 6, et s'étend légèrement en saillie par rapport à cette face inférieure, de façon à venir en contact électrique avec un plot de contact électrique 18 débouchant du pan incliné 11, lorsque la pastille 6 est en place, en butée 12 contre les montants latéraux 13 sur le pan incliné 11. L'extrémité supérieure 19 de la première électrode 7 forme le fond de la chambre de réactif 8 de la pastille 6. La composition réactive contenue dans cette chambre de réactif 8 est donc en contact avec la première électrode 7. La membrane semi-perméable 9 retient la composition réactive au-dessus de l'extrémité supérieure 19 de la première électrode 7, qu'elle vient coiffer. Sur les figures, les échelles relatives en épaisseur ne sont pas respectées, à des fins d'illustration. Ainsi, la chambre de réactif 8 est représentée beaucoup plus épaisse qu'elle ne l'est par rapport à l'épaisseur du corps 16 dans la réalité.

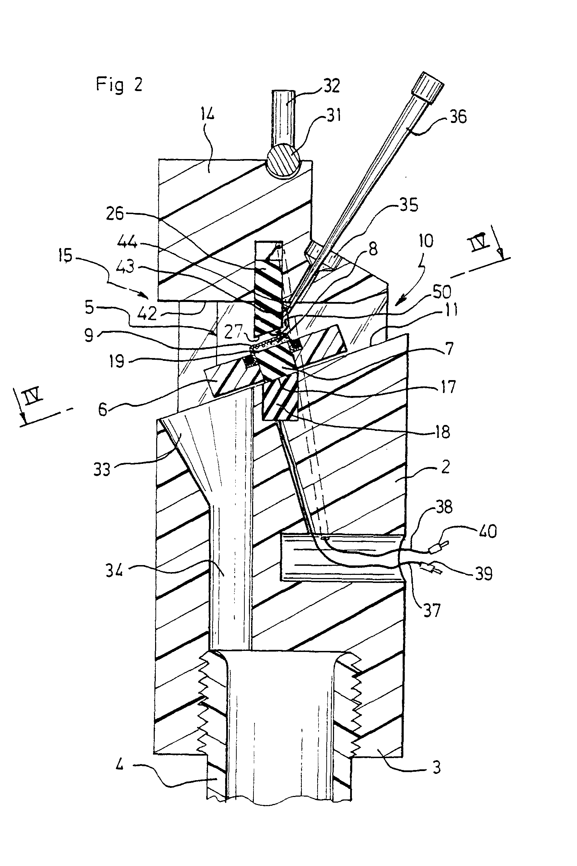

Un premier mode de réalisation d'une pastille 6 selon l'invention est représenté figures 2, 3, 4, 5, 6a à 6c. Dans ce premier mode de réalisation, une gorge 20 est ménagée en creux dans le corps 16 tout autour du fond 19 formé par la première électrode 7, de sorte que cette gorge 20 périphérique est apte à recevoir un joint périphérique 21 bloquant la membrane semi-perméable 9 autour et au-dessus du fond 19. Dans ce premier mode de réalisation, la chambre de réactif 8 est donc intégralement délimitée d'une part par l'extrémité supérieure de la première électrode 7 formant le fond 19, et d'autre part par la membrane semi-perméable 9 qui retient la composition réactive contre ce fond 19 et forme une poche la contenant, en étant bloquée par le joint périphérique 21 placé dans la gorge 20.

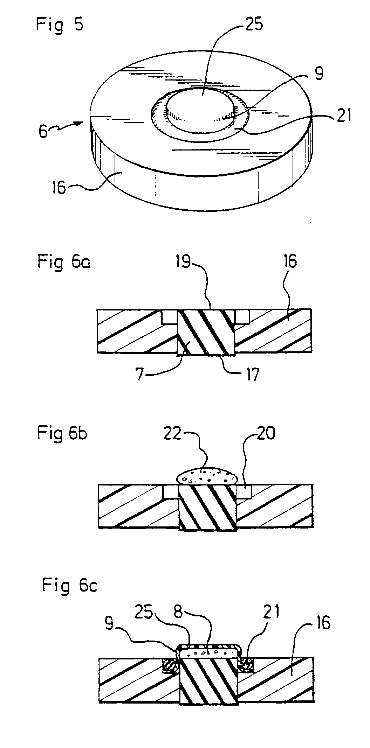

Comme on le voit figures 6a à 6c, pour fabriquer une telle pastille 6, on part du corps 16 dans lequel la première électrode 7 a été engagée, on dépose une quantité prédéterminée sous forme d'une goutte d'une composition réactive 22 (figure 6b) sur l'extrémité supérieure 19 de la première électrode 7, puis on place la membrane semi-perméable 9 au-dessus de cette goutte 22 en la bloquant par rapport à la première électrode 7 grâce au joint 21 engagé dans la gorge 20 (figure 6c).

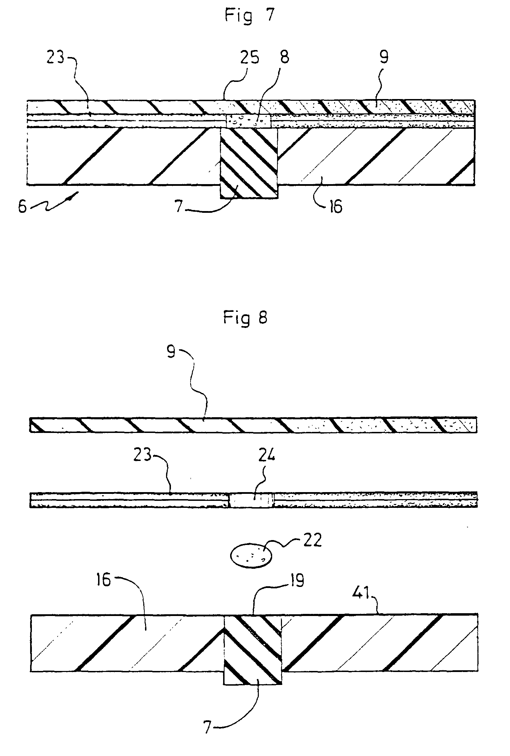

Les figures 7 et 8 représentent un deuxième mode de réalisation de la pastille 6. Dans ce mode de réalisation, la face supérieure 41 du corps 16 de la pastille 6 est plane et vient affleurer l'extrémité supérieure 19 de la première électrode 17. Une feuille d'adhésif double face 23 percée en son centre d'un perçage circulaire 24 de diamètre inférieur ou égal à celui de l'extrémité supérieure 19 de la première électrode 7, est collée sur le corps 16. Le perçage 24 est disposé au-dessus de l'extrémité supérieure 19 de la première électrode 7 et est centré sur cette extrémité supérieure 19. La membrane semi-perméable 9 est en forme de feuille, collée elle-même par dessus la feuille d'adhésif double face 23, de sorte que la membrane semi-perméable 9 referme le perçage 24 qui définit ainsi le volume de la chambre de réactif 8 dans lequel la composition réactive est placée. Pour fabriquer cette pastille, on colle une feuille d'adhésif 23 au-dessus de la membrane semi-perméable 9, puis on dépose une goutte 22 de composition réactive dans le perçage 24, puis on présente le corps 16 doté de la première électrode 7 renversé avec l'extrémité 19 et la face 41 orientées vers le bas, pour le coller sur la feuille d'adhésif 23, ce qui referme le perçage 24. Il suffit ensuite de renverser l'ensemble ainsi formé pour obtenir la pastille 6 de la figure 7. A la place d'une feuille autoadhésive 23, on peut également utiliser une feuille de matière synthétique d'épaisseur prédéterminée collée sur la membrane semi-perméable 9 et sur le corps 16 par une colle appropriée, par exemple de type cyanocrylate.

Dans les deux modes de réalisation représentés, la pastille 6 présente une face externe libre, dite face de réception 25, au moins sensiblement plane s'étendant globalement parallèlement au fond 19 formé par l'extrémité supérieure de la première électrode 7, et au corps 16, et cette face de réception 25 est adaptée pour recevoir une goutte 50 de solution liquide à analyser, cette goutte de solution liquide étant séparée de la composition réactive incluse dans la chambre de réactif 8 par la membrane semi-perméable 9. La face de réception 25 est donc aussi une face de contact, c'est-à-dire qu'elle vient au contact de la solution liquide à analyser. Néanmoins, les échanges ioniques et électroniques à travers la membrane semi-perméable 9 sont possibles, de sorte qu'une réaction d'oxydoréduction se produit, en fonction de la concentration du composé correspondant à l'agent biochimique enzymatique contenu dans la composition réactive.

L'étrier 15 du biocapteur porte également une deuxième électrode 26 disposée à distance de la membrane semi-perméable 9 de façon à venir en contact avec la goutte de solution liquide qui est placée sur la face de réception 25 de la membrane semi-perméable 9, et ce comme représenté figure 2. La deuxième électrode 26 est disposée au-dessus, en regard et à distance de la face de réception 25 d'une pastille 6 en place sur le pan incliné 11, et cette deuxième électrode 26 présente une extrémité libre 27 inférieure comprenant une face plane inclinée s'étendant au moins sensiblement parallèlement à la face de réception 25 qui est elle-même inclinée au moins sensiblement parallèlement au pan incliné 11, c'est-à-dire d'un angle compris entre 0° et 90°, de préférence entre 10° et 75° - notamment de l'ordre de 40°- par rapport à l'horizontale. La distance ménagée entre l'extrémité libre inférieure 27 de la deuxième électrode 26 et la face de réception 25 de la membrane semi-perméable 9 de la pastille 6 est adaptée pour qu'une goutte 50 de solution liquide à analyser soit retenue par capillarité dans l'espace ménagé entre cette extrémité libre 27 et cette face de réception 25. Cette distance dépend donc en particulier du diamètre de la deuxième électrode 26 et de la face de réception 25 qui est de préférence compris entre 1 mm et 10 mm - notamment de l'ordre de 4 mm -, des tensions de surface de la face de réception 25 et de la face libre 27 de la deuxième électrode 26 de la viscosité (donc de la tension de surface) de la solution liquide à analyser, et de l'inclinaison de la face de réception 25. En pratique, avantageusement et selon l'invention, cette distance est inférieure à 8 mm, et supérieure à 1 mm.

Une pastille 6 selon l'invention a typiquement une épaisseur comprise entre 2 mm et 10 mm - notamment de l'ordre de 4 mm -, l'épaisseur de la chambre de réactif 8 étant inférieure à 1 mm, de l'ordre de quelques microns à quelques dixièmes de millimètre. La pastille 6 a avantageusement une dimension en longueur (plus grande dimension perpendiculairement à l'épaisseur, c'est-à-dire dimension radiale, ou diamètre si elle est en forme de disque) comprise entre 5 mm et 50 mm - notamment de l'ordre de 20 mm -. La face libre d'extrémité 27 de la deuxième électrode 26 et la face de réception 25 présentent une dimension moyenne parallèlement au plan de la face de réception 25 (notamment un diamètre dans les modes de réalisation où ces faces sont circulaires) comprise entre 1 mm et 10 mm - notamment de l'ordre de 5 mm -. Les électrodes 7, 26 présentent une dimension radiale moyenne (diamètre s'il s'agit de cylindres de révolution) comprise entre 1 mm et 10 mm - notamment de l'ordre de 4 mm -.

La première électrode 7 est de préférence de forme cylindrique de révolution, la face de réception 25 étant en forme générale de disque. De même, la deuxième électrode 26 est de préférence de forme cylindrique de révolution. Rien n'empêche néanmoins de prévoir, en variantes non représentées, d'autres formes de réalisation (par exemple électrodes 7, 26 prismatiques et face de réception 25 polygonale ...). De même, le corps 16 de la pastille 6 peut être non pas cylindrique, mais prismatique, ou même en forme de coin (avec deux bases non parallèles entre elles) de façon à former ou à participer à l'inclinaison de la face de réception 25 par rapport à l'horizontale (le pan incliné 11 étant moins incliné voire même non incliné).

Par ailleurs, le biocapteur 1 comprend un cavalier 28 métallique comprenant deux branches verticales 29 engagées dans des perçages verticaux correspondant ménagés à travers la tête 14 de l'étrier 15 de façon que les extrémités inférieures 30 de ces branches 29 viennent en appui contre le corps 16 d'une pastille 6 en place sur le pan incliné 11. Le cavalier 28 applique donc, par l'effet de son poids, la pastille 6 contre le pan incliné 11, et la première électrode 7 en contact électrique avec le plot 18. Le cavalier 28 comprend une traverse supérieure 31 reliant les deux branches verticales 29, et dotée d'une extension verticale 32 de manoeuvre permettant à l'utilisateur de soulever le cavalier 28 pour libérer la pastille 6. En variante non représentée, le cavalier 28 peut également être rappelé au contact de la pastille 6 par l'intermédiaire de moyens de rappel à ressort. Les extrémités libres inférieures 30 des branches 29 du cavalier 28 sont de préférence biseautées de façon à s'étendre parallèlement au pan incliné 11 et au corps 16.

Le biocapteur 1 comprend en outre un orifice 33 de récupération de la solution liquide après analyse, et cet orifice 33 est ménagé en partie inférieure du pan incliné 11, de sorte que la solution liquide s'écoule dans l'orifice 33 naturellement par gravité à partir de la face de réception 25, sans venir au contact de la première électrode 7 ou du plot 18 de contact. Cet écoulement peut être provoqué par l'opérateur chassant la goutte (maintenue par capillarité) vers le bas, mécaniquement ou avec un jet d'air comprimé. Un élément absorbant (coton, tissu ...) peut aussi être utilisé. L'orifice 33 communique par l'intermédiaire d'un conduit vertical 34 avec l'extrémité inférieure 3 du bâti 2, de sorte que la composition liquide peut s'écouler dans le récipient de récupération sur lequel le biocapteur 1 est monté.

Par ailleurs, l'étrier 15 du biocapteur 1 comprend un puits d'alimentation 35 en solution liquide, par lequel l'extrémité d'une pipette 36 peut être introduite pour permettre la délivrance d'une dose de solution liquide à analyser correspondant à la goutte 50. Ce puits d'alimentation 35 débouche immédiatement à l'amont et en regard d'une face libre 43 verticale de la deuxième électrode 26, cette face libre 43 étant orientée vers l'amont par rapport à l'inclinaison de la face de réception 25 de la pastille 6, et donc par rapport au sens d'écoulement de la solution liquide sur cette face de réception 25.

La deuxième électrode 26 s'étend au-dessus et en regard de la face de réception 25. Elle est portée par l'étrier 15 du bâti 2 qui présente une face inférieure 42 libre orientée vers le bas, s'étendant au-dessus et à distance du pan incliné 11 pour former le compartiment de réception de la pastille 6. La deuxième électrode 26 s'étend en saillie vers le bas à partir de cette face inférieure 42 de l'étrier 15 de façon à présenter ladite face libre 43, contre laquelle l'extrémité 44 d'une pipette 36 engagée dans le puits d'alimentation 35 peut être placée pour y déposer une dose (goutte 50) de solution liquide. La goutte 50 de solution liquide ainsi déposée s'écoule vers le bas par gravité pour venir combler l'espace entre la deuxième électrode 26 et la face de réception 25, et elle y reste maintenue par capillarité. Si un surplus de solution liquide est déposé, ce dernier va s'écouler par gravité vers le bas sur la pastille 6 inclinée, puis dans l'orifice 33 de récupération, et ce grâce à l'inclinaison de la face de réception 25 et de la pastille 6 qui est adaptée à cet effet.

Le plot de contact 18 et la deuxième électrode 26 sont reliés à un circuit électrique extérieur par un fil conducteur 37, respectivement 38, débouchant à l'exterieur du bâti 2. Chacun de ces fils 37, 38 est passé dans un conduit ménage à travers le bâti 2 pour déboucher à l'extérieur du bâti 2. Chaque fil 37, 38 présente, à son extrémité libre extérieure, un connecteur 39, respectivement 40, pour sa connexion à un circuit électrique extérieur auquel est fourni le courant électrique correspondant à la mesure de concentration du composé chimique dans la solution liquide a analyser, notamment pour la mesure de l'intensité du courant électrique dans le cas d'électrodes 7, 26 du type ampèromètrique.

La première électrode 7 portée par une pastille 6 est reliée au fil 37 par l'intermédiaire du plot de contact 18 auquel le fil 37 est soudé. Par contre, le fil 38 de la deuxième électrode 26 est directement soudé sur cette deuxième électrode 26.

La membrane semi-perméable 9 utilisée dans une pastille 6 de l'invention, peut être une membrane semi-perméable en cellophane telle qu'une membrane de dialyse les électrodes 7, 26 peuvent être réalisées entièrement en or ou en platine, ou en alliage métallique revêtu d'or ou de platine, ou en tout autre matériau électriquement conducteur résistant à l'électrolyse.

La pastille 6 est immédiatement amovible par rapport au bâti 2 par simple manoeuvre manuelle sans outil. Pour enlever une pastille 6 en place dans la zone d'analyse 5, il suffit en effet de soulever le cavalier 28 en tirant vers le haut sur l'extension 32 de manoeuvre, et d'extraire la pastille 6 par la fente 10 en la faisant glisser vers le haut sur le pan incliné 11. Pour mettre en place une nouvelle pastille 6, on l'introduit dans la fente 10 jusqu'à ce qu'elle vienne en butée 12 inférieure contre les montants latéraux 13, puis on relâche le cavalier 28 dont les branches 29 viennent au contact du corps 16 de la pastille 6 en la plaquant sur le pan incliné 11. La pastille 6 est ainsi automatiquement précisément placée par rapport au plot de contact 18 et par rapport à la deuxième électrode 26. On peut immédiatement injecter une goutte d'une dose de solution liquide à analyser avec une pipette 36 comme représenté figure 2.

Ce mode de réalisation de l'invention est notamment applicable avantageusement à la réalisation d'un biocapteur utilisable dans tous les cas où il est nécessaire de confiner une composition liquide biochimique réactive dans une chambre de réactif 8 séparée d'une solution liquide à analyser, par une membrane semi-perméable 9, une électrode étant en contact avec la composition liquide à analyser, tandis que l'autre électrode est en contact avec la solution réactive. L'invention est en particulier applicable pour les mesures de la concentration d'un composé chimique dans une solution liquide aqueuse d'origine naturelle ou artificielle, avec une composition réactive enzymatique. La valeur de la concentration est obtenue par la mesure du courant circulant entre les électrodes suite à la réaction chimique enzymatique d'oxydoréduction générée dans la solution liquide par la composition réactive enzymatique. On peut ainsi notamment mesurer précisément et rapidement les concentrations en glucose ou en lactate.

L'invention s'applique néanmoins tout aussi bien à la mesure de tout autre composé chimique, et notamment pour la détection et la mesure de concentrations de différents composés dans les vins, les substances agroalimentaires, les liquides physiologiques. Ainsi, l'invention peut faire l'objet de très nombreuses applications : dosage du glucose, du lactate, de l'urée, du cholestérol, ou d'autres métabolites (hormones, anti-corps ...), d'alcools ou des drogues illicites, et ce par prélèvement d'une solution liquide, sanguine ou autre d'un organisme humain ou animal ; dosage de composés tel que les sucres, les acides aminés, le glutamate, le lactate, ... dans les produits finis ou les procédés d'élaboration des produits agroalimentaires tels que le pain, le lait, les laitages, les vins, les bières ... ; mesure de la maturité des fruits ; mesure de la fraîcheur des poissons et des viandes ; détermination de la contamination bactérienne d'un produit agroalimentaire ; dosage d'agents toxiques dans les solutions liquides industrielles ou naturelles (pesticides, fongicides, nitrates, phénols, organochlorés ou organophosphorés, liants métalliques ; mesure de la demande biologique en oxygène, détection de la contamination organique des eaux ...)...

Par ailleurs, l'invention peut faire l'objet de nombreuses variantes par rapport aux modes de réalisation décrits et représentés figures 1 à 8. En particulier, la forme, les dimensions, et le matériau constitutif du biocapteur 1 et des pastilles 6 peuvent varier dans une large mesure. Le bâti 2 peut être lui-même réalisé en une seule pièce moulée et/ou usinée, ou de préférence en plusieurs pièces moulées et/ou usinées assemblées les unes aux autres ultérieurement.

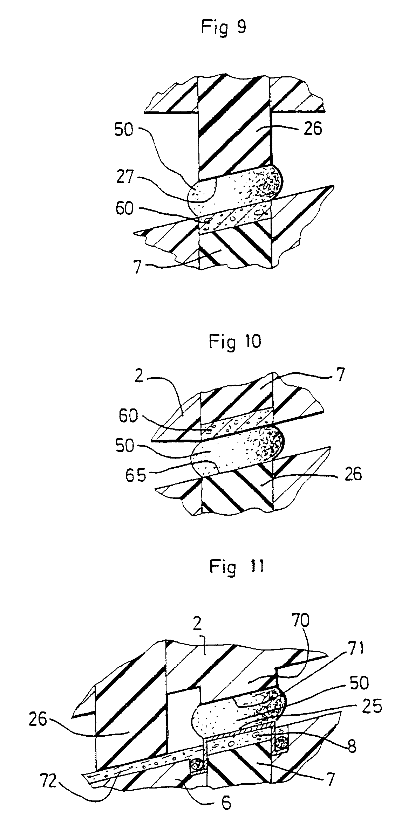

Comme on le voit figure 9, la pastille peut être formée d'une pastille 60 solide ou semi-solide de composition réactive immobilisée sous forme de gel, entièrement formée de la chambre de réactif 8, et qui peut être montée amovible dans un logement du bâti 2.

La variante de la figure 10 montre que le système peut aussi bien être renversé, avec la chambre de réactif formée de la pastille 60 et la première électrode 7 placées au-dessus, la deuxième électrode 26 étant placée en dessous, faisant office d'organe de retenue de la goutte 50, et présentant une face de réception 65 de la goutte 50.

Le système peut être aussi placé dans toute autre position.

Par ailleurs, comme on le voit figure 11, la goutte 50 peut être maintenue entre la chambre de réactif 8 et une pièce 70, distincte de la deuxième électrode 26 (et aussi bien sûr de la première électrode 7). Cette pièce 70 présente une face 71 en regard de la face libre 25 de contact de la chambre de réactif 8. La deuxième électrode 26 peut alors être adaptée pour venir au contact d'un canal 72 rempli d'eau ou de solution conductrice ménagé en creux dans la pastille 6 vers l'extérieur radialement à partir de la membrane 9. Ce canal 72 peut être suffisamment fin pour retenir l'eau ou la solution conductrice par capillarité. En variante non représentée, il peut être placé sur le côté de façon à s'étendre latéralement horizontalement même lorsque la face libre 25 de la chambre de réactif 8 et celle 71 de la pièce 70 sont inclinées.

Ces différentes variantes peuvent être combinées.

## Revendications

1. Biocapteur électrochimique de mesure de la concentration d'un composé dans une solution liquide, comprenant :
une chambre de réactif (8) adaptée pour renfermer une quantité de composition biochimique, dite composition réactive, et présentant une face externe libre, dite face de contact (25), apte à être placée au contact de la solution liquide qui est ainsi elle-même en contact avec la composition réactive,
une première (7) et une deuxième (26) électrodes adaptées pour être électriquement reliées à la solution liquide et permettre une mesure électrique entre elles,
**caractérisé en ce qu'**il comprend, en regard de la face de contact (25), un organe de retenue (26, 70), la forme de cet organe de retenue (26, 70) et la distance entre la face de contact (25) et l'organe de retenue (26, 70) étant adaptées pour qu'une goutte (50) de solution liquide placée entre la face de contact (25) et cet organe de retenue (26, 70) soit retenue et maintenue entre eux par capillarité.

2. Biocapteur selon la revendication 1, **caractérisé en ce que** la première électrode (7) est électriquement reliée à la chambre de réactif (8), à l'opposé de la face de contact (25), et **en ce que** la deuxième électrode (26) est adaptée pour être électriquement reliée avec la goutte (50) de solution liquide du côté de la face de contact (25).

3. Biocapteur selon l'une des revendications précédentes, **caractérisé en ce que** la deuxième électrode (26) est adaptée pour être directement au contact de la goutte (50) de solution liquide.

4. Biocapteur selon l'une des revendications 1 ou 2, **caractérisé en ce que** la deuxième électrode est adaptée pour être électriquement reliée à la goutte (50) de solution liquide par au moins un élément intermédiaire électriquement conducteur.

5. Biocapteur selon l'une des revendications précédentes, **caractérisé en ce que** l'organe de retenue (26, 70) est formé de la deuxième électrode (26) directement en contact avec la goutte (50) de solution liquide.

6. Biocapteur selon l'une des revendications 1 à 4, **caractérisé en ce que** l'organe de retenue est un organe (70) spécifique distinct d'une électrode, et **en ce que** les deux électrodes (26, 7) sont électriquement reliées à la chambre de réactif (8).

7. Biocapteur selon l'une des revendications précédentes, **caractérisé en ce que** l'organe de retenue (26, 70) présente une face (27, 71) en regard de la face de contact (25).

8. Biocapteur selon l'une des revendications précédentes, **caractérisé en ce que** l'ensemble formé de l'organe de retenue (26, 70) et de la face de contact (25) présente une face, orientée vers le haut, dite face de réception (25, 65), et **en ce que** la face de réception (25, 65) présente un angle d'inclinaison par rapport à l'horizontale, qui est supérieur à 0° et inférieur à 90° -notamment de l'ordre de 40°-.

9. Biocapteur selon les revendications 7 et 8, **caractérisé en ce que** la face de réception est la face (65) de l'organe de retenue (26).

10. Biocapteur selon la revendication 8, **caractérisé en ce que** la face de réception est la face de contact (25) de la chambre de réactif (8).

11. Biocapteur selon l'une des revendications précédentes, **caractérisé en ce que** la distance entre l'organe de retenue (26, 70) et la face de contact (25) est inférieure à 8 mm.

12. Biocapteur selon l'une des revendications précédentes, dans lequel la chambre de réactif (8) est adaptée pour renfermer une quantité de composition réactive liquide, et comporte une membrane semi-perméable (9) refermant la chambre de réactif (8) de façon à y retenir la composition réactive, cette membrane semi-perméable (9) présentant une face externe libre, formant ladite face de contact (25), apte à être placée au contact de la solution liquide séparée de la composition réactive par la membrane semi-perméable (9),
**caractérisé en ce qu'**il comporte deux pièces distinctes :
- une pastille (6) comprenant la première électrode (7), la chambre de réactif (8) renfermant la composition réactive liquide, et la membrane semi-perméable (9),
- un bâti (2) comprenant des moyens (11, 12) de réception d'une pastille (6), des moyens (18, 37, 39) de connexion électrique, avec un circuit électrique extérieur, de la première électrode (7), d'une pastille (6) en place dans les moyens (11, 12) de réception, ce bâti (2) portant la deuxième électrode (26) à distance de la membrane semi-perméable (9) d'une pastille (6) en place dans les moyens (11, 12) de réception, et comprenant des moyens (38, 40) de liaison électrique de la deuxième électrode (26) avec le circuit électrique extérieur.

13. Biocapteur selon les revendications 4, 7, 8, 10 et 12, **caractérisé en ce que** la deuxième électrode (26) comprend une extrémité libre (27) s'étendant à distance et en regard de ladite face de réception (25) de la membrane semi-perméable (9) d'une pastille (6), et cette extrémité libre (27) présente une face inclinée s'étendant au moins sensiblement parallèlement à la face de réception (25) inclinée.

14. Biocapteur selon les revendications 4, 7, 8, 10 et 12, ou selon la revendication 13, **caractérisé en ce que** la deuxième électrode (26) s'étend au-dessus de la face de réception (25) et en saillie vers le bas par rapport à une face (42) du bâti (2) orientée vers le bas, de sorte que cette deuxième électrode (26) présente au moins une face libre (43) s'étendant vers le bas et orientée vers l'amont par rapport à l'inclinaison de la face de réception (25) de la pastille (6), et **en ce qu'**il comprend un puits d'alimentation (35) en solution liquide débouchant immédiatement à l'amont et en regard de la face libre (43) de la deuxième électrode (26), de sorte que la solution liquide est alimentée et déposée sur cette face libre (43) pour s'écouler vers le bas le long de la deuxième électrode (26) jusqu'à venir dans l'interstice séparant la deuxième électrode (26) et la face de réception (25) inclinée.

15. Biocapteur selon l'une des revendications 12 à 14, **caractérisé en ce que** les moyens (11, 12) de réception d'une pastille comprennent un pan incliné (11) orienté vers le haut, et des moyens (12) formant une butée de réception d'une pastille (6) en position basse extrême sur le pan incliné (11), **en ce que** les moyens (18, 37, 39) de connexion électrique comportent un plot (18) de contact électrique débouchant du pan incliné (11) et adapté pour venir en liaison électrique avec une portion (17) conductrice inférieure de la pastille (6) en place et en butée sur le pan incliné (11), cette portion conductrice (17) étant en liaison électrique avec la première électrode (7), et **en ce que** le bâti (2) comprend un étrier (15) portant la deuxième électrode (26) au-dessus, en regard et à distance de ladite face de réception (25) de la membrane semi-perméable (9) d'une pastille (6) en place et en butée sur le pan incliné (11).

16. Biocapteur selon la revendication 15, **caractérisé en ce qu'**il comprend des moyens (28) pour plaquer la pastille (6) contre le pan incliné (11).

17. Biocapteur selon l'une des revendications 12 à 16, **caractérisé en ce que** le bâti (2) comprend un orifice (33) de récupération de la solution liquide disposé de façon à pouvoir récupérer la solution liquide s'écoulant de la face de réception (25) et communiquant avec une extrémité inférieure (3) du bâti.

18. Biocapteur selon l'une des revendications 12 à 17, **caractérisé en ce qu'**il comprend des moyens (3) de montage du bâti (2) sur un récipient de récupération de la solution liquide.

19. Pastille de biocapteur électrochimique de mesure de la concentration d'un composé dans une solution liquide, **caractérisée en ce qu'**elle comprend :
- une chambre de réactif (8) renfermant une quantité de composition biochimique liquide, dite composition réactive, et comportant une membrane semi-perméable (9) refermant la chambre de réactif (8) de façon à y retenir la composition réactive, cette membrane semi-perméable (9) présentant une face externe libre, dite face de réception (25), apte à recevoir une goutte de solution liquide séparée de la composition réactive par la membrane semi-perméable (9),
- une électrode, dite première électrode (7), placée au contact électrique de la composition réactive contenue dans la chambre de réactif (8), et des moyens (17) de connexion électrique de cette première électrode (7) avec un circuit électrique extérieur à la pastille (6).

20. Pastille selon la revendication 19, **caractérisée en ce que** la première électrode (7) présente une extrémité (19) débouchant dans la chambre de réactif (8) à l'opposé d'une portion de la membrane semi-perméable (9) formant ladite face de réception (25).

21. Pastille selon l'une des revendications 19 ou 20, **caractérisée en ce que** la première électrode (7) forme un fond (19) de la chambre de réactif (8) refermée, à l'opposé de ce fond (19), par la membrane semi-perméable (9).

22. Pastille selon la revendication 21, **caractérisée en ce que** la chambre de reactif (8) est délimitée par le fond (19) formé par la première électrode (7) et par la membrane semi-perméable (9) s'étendant à partir du fond (19) et au-dessus du fond (19).

23. Pastille selon les revendications 21 et 22, **caractérisée en ce qu'**elle présente une gorge (20) en creux autour du fond (19) formé par la première électrode (7), cette gorge (20) étant adaptée pour recevoir un joint périphérique (21) bloquant la membrane semi-perméable (9) autour et au-dessus de ce fond (19).

24. Pastille selon l'une des revendications 19 à 23, **caractérisée en ce que** la première électrode (7) s'étend de façon à présenter une portion (17) débouchant a l'extérieur de la pastille (6) pour former des moyens (17) de connexion électrique avec un circuit électrique extérieur.

25. Pastille selon l'une des revendications 19 à 24, **caractérisée en ce qu'**elle comprend un corps (16) de matière synthétique électriquement isolante en forme générale de plaquette, et **en ce que** la première électrode (7) traverse l'épaisseur de ce corps (16).

26. Pastille selon l'une des revendications 19 à 25, **caractérisée en ce qu'**elle est en forme générale de disque.

27. Pastille selon l'une des revendications 19 à 26, **caractérisée en ce qu'**elle présente une épaisseur comprise entre 2 mm et 10 mm et une dimension en longueur comprise entre 5 mm et 50 mm.

28. Pastille selon l'une des revendications 19 à 27, **caractérisée en ce que** la première électrode (7) présente une dimension radiale moyenne comprise entre 1 mm et 10 mm - notamment de l'ordre de 4 mm -.

29. Pastille selon l'une des revendications 19 à 28, **caractérisée en ce que** la composition réactive est une solution aqueuse enzymatique.

## Patentansprüche

1. Elektrochemischer Biosensor zum Messen der Konzentration einer Verbindung in einer flüssigen Lösung, der Folgendes umfasst:
- eine Reaktionsmittelkammer (8) zum Aufnehmen einer Menge an biochemischer Zusammensetzung, reaktive Zusammensetzung genannt, und mit einer freien Außenfläche, Kontaktfläche (25) genannt, die mit der flüssigen Lösung in Kontakt gebracht werden kann, die somit wiederum mit der reaktiven Zusammensetzung in Kontakt ist,
- eine erste (7) und eine zweite (26) Elektrode, die so gestaltet sind, dass sie elektrisch mit der flüssigen Lösung in Verbindung gebracht werden und eine elektrische Messung dazwischen ermöglichen können,
**dadurch gekennzeichnet, dass** er ein der Kontaktfläche (25) zugewandtes Halteelement (26, 70) umfasst, dessen Form und dessen Abstand zur Kontaktfläche (25) derart sind, dass ein Tropfen (50) der flüssigen Lösung zwischen der Kontaktfläche (25) und diesem Halteelement (26, 70) durch Kapillarwirkung dazwischen gehalten wird.

2. Biosensor nach Anspruch 1, **dadurch gekennzeichnet, dass** die erste Elektrode (7) elektrisch mit der Reaktionsmittelkammer (8) gegenüber der Kontaktfläche (25) verbunden ist, und dadurch, dass die zweite Elektrode (26) so gestaltet ist, dass sie mit dem Tropfen (50) der flüssigen Lösung auf der Seite der Kontaktfläche (25) verbunden ist.

3. Biosensor nach einem der vorherigen Ansprüche, **dadurch gekennzeichnet, dass** die zweite Elektrode (26) so gestaltet ist, dass sie direkt mit dem Tropfen (50) der flüssigen Lösung in Kontakt ist.

4. Biosensor nach Anspruch 1 oder 2, **dadurch gekennzeichnet, dass** die zweite Elektrode so gestaltet ist, dass sie durch wenigstens ein leitendes Zwischenelement mit dem Tropfen (50) der flüssigen Lösung elektrisch verbunden ist.

5. Biosensor nach einem der vorherigen Ansprüche, **dadurch gekennzeichnet, dass** das Halteelement (26, 70) von der zweiten Elektrode (26) direkt in Kontakt mit dem Tropfen (50) der flüssigen Lösung gebildet wird.

6. Biosensor nach einem der Ansprüche 1 bis 4, **dadurch gekennzeichnet, dass** das Halteelement ein spezifisches Element (70) ist, das sich von einer Elektrode unterscheidet, und dadurch, dass die beiden Elektroden (26, 7) elektrisch mit der Reaktionsmittelkammer (8) verbunden sind.

7. Biosensor nach einem der vorherigen Ansprüche, **dadurch gekennzeichnet, dass** das Halteelement (26, 70) eine der Kontaktfläche (25) zugewandte Fläche (27, 71) hat.

8. Biosensor nach einem der vorherigen Ansprüche, **dadurch gekennzeichnet, dass** die aus dem Halteelement (26, 70) und der Kontaktfläche (25) gebildete Baugruppe eine nach oben ausgerichtete Fläche, Aufnahmefläche (25, 65) genannt, aufweist, und dadurch, dass die Aufnahmefläche (25, 65) einen Neigungswinkel in Bezug auf die Horizontale hat, der größer als 0° und kleiner als 90° ist und insbesondere etwa 40° beträgt.

9. Biosensor nach den Ansprüchen 7 und 8, **dadurch gekennzeichnet, dass** die Aufnahmefläche die Fläche (65) des Halteelementes (26) ist.

10. Biosensor nach Anspruch 8, **dadurch gekennzeichnet, dass** die Aufnahmefläche die Kontaktfläche (25) der Reaktionsmittelkammer (8) ist.

11. Biosensor nach einem der vorherigen Ansprüche, **dadurch gekennzeichnet, dass** der Abstand zwischen dem Halteelement (26, 70) und der Kontaktfläche (25) geringer als 8 mm ist.

12. Biosensor nach einem der vorherigen Ansprüche, bei dem die Reaktionsmittelkammer (8) eine Menge an flüssiger reaktiver Zusammensetzung aufnimmt und eine teildurchlässige Membran (9) aufweist, die die Reaktionsmittelkammer (8) verschließt, so dass die reaktive Zusammensetzung darin gehalten werden kann, wobei diese teildurchlässige Membran (9) eine freie Außenfläche hat, die die genannte Kontaktfläche (25) bildet und die in Kontakt mit der flüssigen Lösung gebracht werden kann, die von der reaktiven Zusammensetzung durch die teildurchlässige Membran (9) getrennt ist, **dadurch gekennzeichnet, dass** sie zwei getrennte Teile aufweist:
- einen Chip (6), der die erste Elektrode (7), die die flüssige reaktive Zusammensetzung aufnehmende Reaktionsmittelkammer (8) und die teildurchlässige Membran (9) umfasst,
- ein Gehäuse (2), umfassend Mittel (11, 12) zur Aufnahme eines Chips (6), Mittel (18, 37, 39) zum elektrischen Verbinden, mit einer externen elektrischen Schaltung, der ersten Elektrode (7), eines Chips (6) am Ort in den Aufnahmemitteln (11, 12), wobei dieses Gehäuse (2) die zweite Elektrode (26) in einem Abstand von der teildurchlässigen Membran (9) eines Chips (6) am Ort in den Aufnahmemitteln (11, 12) trägt, und umfassend Mittel (38, 40) zum elektrischen Verbinden der zweiten Elektrode (26) mit der externen elektrischen Schaltung.

13. Biosensor nach den Ansprüchen 4, 7, 8, 10 und 12, **dadurch gekennzeichnet, dass** die zweite Elektrode (26) ein freies Ende (27) aufweist, das in einem Abstand von der genannten Aufnahmefläche (25) der teildurchlässigen Membran (9) eines Chips (6) verläuft und dieser zugewandt ist, und dieses freie Ende (27) eine geneigte Fläche hat, die wenigstens im Wesentlichen parallel zu der geneigten Aufnahmefläche (25) verläuft.

14. Biosensor nach den Ansprüchen 4, 7, 8, 10 und 12 oder nach Anspruch 13, **dadurch gekennzeichnet, dass** die zweite Elektrode (26) über der Aufnahmefläche (25) und mit einem Vorsprung nach unten in Bezug auf eine Fläche (42) des nach unten ausgerichteten Gehäuses (2) verläuft, so dass diese zweite Elektrode (26) wenigstens eine freie Fläche (43) hat, die abwärts verläuft und stromaufwärts in Bezug auf die Neigung der Aufnahmefläche (25) des Chips (6) ausgerichtet ist, und dadurch, dass der Biosensor einen Bunker (35) zum Zuführen von flüssiger Lösung umfasst, der unmittelbar oberhalb der freien Fläche (43) der zweiten Elektrode (26) austritt und dieser zugewandt ist, so dass die flüssige Lösung dieser freien Fläche (43) zugeführt und dort deponiert wird, so dass sie entlang der zweiten Elektrode (26) abwärts fließt, bis sie an dem Spalt ankommt, der die zweite Elektrode (26) von der geneigten Aufnahmefläche (25) trennt.

15. Biosensor nach einem der Ansprüche 12 bis 14, **dadurch gekennzeichnet, dass** die Mittel (11, 12) zum Aufnehmen eines Chips eine nach oben ausgerichtete geneigte Fläche (11) und Mittel (12) umfasst, die einen Anschlag zur Aufnahme eines Chips (6) in der unteren Endposition auf der geneigten Fläche (11) bilden, und dadurch, dass die Mittel (18, 37, 39) zum elektrischen Verbinden einen elektrischen Kontaktbolzen (18) aufweisen, der von der geneigten Fläche (11) austritt und so gestaltet ist, dass er in elektrischer Verbindung mit einem unteren leitenden Teil (17) des Chips (6) an der geneigten Fläche (11) zur Anlage kommt, wobei dieser leitende Abschnitt (17) in elektrischer Verbindung mit der ersten Elektrode (7) ist, und dadurch, dass das Gehäuse (2) einen Bügel (15) umfasst, der die zweite Elektrode (26) über, in einem Abstand von der genannten Aufnahmefläche (25) der teildurchlässigen Membran (9) eines Chips (6) und dieser zugewandt in Anlage an der geneigten Fläche (11) trägt.

16. Biosensor nach Anspruch 15, **dadurch gekennzeichnet, dass** er Mittel (28) zum Drücken des Chips (6) gegen die geneigte Fläche (11) umfasst.

17. Biosensor nach einem der Ansprüche 12 bis 16, **dadurch gekennzeichnet, dass** das Gehäuse (2) einen Auslass (33) zum Zurückgewinnen der flüssigen Lösung umfasst, der so angeordnet ist, dass die an der Aufnahmefläche (25) herablaufende flüssige Lösung, die mit einem unteren Ende (3) des Gehäuses in Verbindung ist, zurückgewonnen werden kann.

18. Biosensor nach einem der Ansprüche 12 bis 17, **dadurch gekennzeichnet, dass** er Mittel (3) zum Montieren des Gehäuses (2) an einer Fassung zum Zurückgewinnen der flüssigen Lösung umfasst.

19. Elektrochemischer Biosensor-Chip zum Messen der Konzentration einer Verbindung in einer flüssigen Lösung, **dadurch gekennzeichnet, dass** er Folgendes umfasst:
- eine Reaktionsmittelkammer (8) zur Aufnahme einer Menge von biochemischer flüssiger Zusammensetzung, reaktive Zusammensetzung genannt, und mit einer teildurchlässigen Membran (9), die die Reaktionsmittelkammer (8) verschließt, um die reaktive Zusammensetzung darin aufzunehmen, wobei diese teildurchlässige Membran (9) eine freie Außenfläche, Aufnahmefläche (25) genannt, hat, die einen Tropfen der flüssigen Lösung aufnehmen kann, die von der reaktiven Zusammensetzung durch die teildurchlässige Membran (9) getrennt ist,
- eine Elektrode, erste Elektrode (7) genannt, die sich in elektrischem Kontakt mit der reaktiven Zusammensetzung in der Reaktionsmittelkammer (8) befindet, und Mittel (17) zum elektronischen Verbinden dieser ersten Elektrode (7) mit einer elektronischen Schaltung außerhalb des Chips (6).

20. Chip nach Anspruch 19, **dadurch gekennzeichnet, dass** die erste Elektrode (7) ein Ende (19) hat, das in die Reaktionsmittelkammer (8) gegenüber einem Abschnitt der die genannte Aufnahmefläche (25) bildenden teildurchlässigen Membran (9) austritt.

21. Chip nach Anspruch 19 oder 20, **dadurch gekennzeichnet, dass** die erste Elektrode (7) einen Boden (19) der Reaktionsmittelkammer (8) bildet, die gegenüber diesem Boden (19) durch die teildurchlässige Membran (9) geschlossen wird.

22. Chip nach Anspruch 21, **dadurch gekennzeichnet, dass** die Reaktionsmittelkammer (8) durch den von der ersten Elektrode (7) gebildeten Boden (19) und durch die teildurchlässige Membran (9) begrenzt wird, die vom Boden (19) und über dem Boden (19) verläuft.

23. Chip nach Anspruch 21 und 22, **dadurch gekennzeichnet, dass** er eine eingelassene Rille (20) um den von der ersten Elektrode (7) gebildeten Boden (19) hat, wobei diese Rille (20) die Aufgabe hat, eine Umfangsdichtung (21) aufzunehmen, die die teildurchlässige Membran (9) um diesen und über diesem Boden (19) blockiert.

24. Chip nach einem der Ansprüche 19 bis 23, **dadurch gekennzeichnet, dass** die erste Elektrode (7) so verläuft, dass sie einen Abschnitt (17) hat, der aus dem Chip (6) austritt, um Mittel (17) für eine elektrische Verbindung mit einer externen elektrischen Schaltung zu bilden.

25. Chip nach einem der Ansprüche 19 bis 24, **dadurch gekennzeichnet, dass** er einen Körper (16) aus einem elektrisch isolierenden synthetischen Material in der allgemeinen Form einer kleinen Platte umfasst, und wobei die erste Elektrode (7) die Dicke dieses Körpers (16) überquert.

26. Chip nach einem der Ansprüche 19 bis 25, **dadurch gekennzeichnet, dass** er die allgemeine Form einer Scheibe hat.

27. Chip nach einem der Ansprüche 19 bis 26, **dadurch gekennzeichnet, dass** er eine Dicke von 2 mm bis 10 mm und eine Länge von 5 mm bis 50 mm hat.

28. Chip nach einem der Ansprüche 19 bis 27, **dadurch gekennzeichnet, dass** die erste Elektrode (7) eine mittlere radiale Abmessung zwischen 1 mm und 10 mm, insbesondere von etwa 4 mm hat.

29. Chip nach einem der Ansprüche 19 bis 28, **dadurch gekennzeichnet, dass** die reaktive Zusammensetzung eine enzymatische wässrige Lösung ist.

## Claims

1. Electrochemical biosensor for measurement of the concentration of a compound in a liquid solution, comprising:
- a reagent chamber (8) adapted to enclose a quantity of biochemical composition, termed reactive composition, and having a free outer face, termed contact face (25), capable of being placed in contact with the liquid solution which is thus itself in contact with the reactive composition,
- a first electrode (7) and a second electrode (26) which are adapted to be electrically connected to the liquid solution and to enable electrical measurement between them,
**characterised in that** it comprises, facing the contact face (25), a retaining member (26, 70), the shape of this retaining member (26, 70) and the distance between the contact face (25) and the retaining member (26, 70) being adapted so that a drop (50) of liquid solution placed between the contact face (25) and this retaining member (26, 70) is retained and maintained between them by capillary action.

2. Biosensor according to claim 1, **characterised in that** the first electrode (7) is electrically connected to the reagent chamber (8), opposite the contact face (25), and **in that** the second electrode (26) is adapted to be electrically connected with the drop (50) of liquid solution on the side of the contact face (25).

3. Biosensor according to one of the preceding claims, **characterised in that** the second electrode (26) is adapted to be directly in contact with the drop (50) of liquid solution.

4. Biosensor according to one of claims 1 and 2, **characterised in that** the second electrode is adapted to be electrically connected to the drop (50) of liquid solution by at least one electrically conductive intermediate element.

5. Biosensor according to one of the preceding claims, **characterised in that** the retaining member (26, 70) is formed of the second electrode (26) directly in contact with the drop (50) of liquid solution.

6. Biosensor according to one of claims 1 to 4, **characterised in that** the retaining member is a specific member (70) distinct from an electrode, and **in that** the two electrodes (26, 7) are electrically connected to the reagent chamber (8).

7. Biosensor according to one of the preceding claims, **characterised in that** the retaining member (26, 70) has a face (27, 71) facing the contact face (25).

8. Biosensor according to one of the preceding claims, **characterised in that** the assembly formed of the retaining member (26, 70) and of the contact face (25) has a face oriented upwards, termed receiving face (25, 65), and **in that** the receiving face (25, 65) has an angle of inclination with respect to the horizontal which is greater than 0° and less than 90° - in particular of the order of 40°.

9. Biosensor according to claims 7 and 8, **characterised in that** the receiving face is the face (65) of the retaining member (26).

10. Biosensor according to claim 8, **characterised in that** the receiving face is the contact face (25) of the reagent chamber (8).

11. Biosensor according to one of the preceding claims, **characterised in that** the distance between the retaining member (26, 70) and the contact face (25) is less than 8 mm.

12. Biosensor according to one of the preceding claims, in which the reagent chamber (8) is adapted to enclose a quantity of liquid reactive composition, and has a semi-permeable membrane (9) closing the reagent chamber (8) so as to retain therein the reactive composition, this semi-permeable membrane (9) having a free outer face, forming the said contact face (25), capable of being placed in contact with the liquid solution separated from the reactive composition by the semi-permeable membrane (9), **characterised in that** it has two distinct parts:
- a chip (6) comprising the first electrode (7), the reagent chamber (8) enclosing the liquid reactive composition, and the semi-permeable membrane (9),
- a mount (2) comprising means (11, 12) for receiving a chip (6), means (18, 37, 39) for electrical connection, with an external electrical circuit, of the first electrode (7), of a chip (6) in place in the receiving means (11, 12), this mount (2) carrying the second electrode (26) at a distance from the semi-permeable membrane (9) of a chip (6) in place in the receiving means (11, 12), and comprising means (38, 40) for electrical connection of the second electrode (26) with the external electrical circuit.

13. Biosensor according to claims 4, 7, 8, 10 and 12, **characterised in that** the second electrode (26) comprises a free end (27) extending at a distance from and facing the said receiving face (25) of the semi-permeable membrane (9) of a chip (6), and this free end (27) has an inclined face extending at least substantially parallel to the inclined receiving face (25).

14. Biosensor according to claims 4, 7, 8, 10 and 12, or according to claim 13, **characterised in that** the second electrode (26) extends above the receiving face (25) and with a projection downwards with respect to a face (42) of the mount (2) oriented downwards, so that this second electrode (26) has at least one free face (43) extending downwards and oriented upstream with respect to the inclination of the receiving face (25) of the chip (6), and **in that** it comprises a liquid-solution supply shaft (35) emerging immediately upstream of and facing the free face (43) of the second electrode (26), so that the liquid solution is supplied and deposited on this free face (43) to flow downwards along the second electrode (26) until it comes into the gap separating the second electrode (26) and the inclined receiving face (25).

15. Biosensor according to one of claims 12 to 14, **characterised in that** the means (11, 12) for receiving a chip comprise an inclined face (11) oriented upwards, and means (12) forming a stop for receiving a chip (6) in the bottom end position on the inclined face (11), **in that** the means (18, 37, 39) for electrical connection have an electrical contact stud (18) emerging from the inclined face (11) and adapted to come into electrical connection with a lower conductive portion (17) of the chip (6) in place and in abutment on the inclined face (11), this conductive portion (17) being in electrical connection with the first electrode (7), and **in that** the mount (2) comprises a frame (15) carrying the second electrode (26) above, facing and at a distance from the said receiving face (25) of the semi-permeable membrane (9) of a chip (6) in place and in abutment on the inclined face (11).

16. Biosensor according to claim 15, **characterised in that** it comprises means (28) for pressing the chip (6) against the inclined face (11).

17. Biosensor according to one of claims 12 to 16, **characterised in that** the mount (2) comprises an orifice (33) for recovery of the liquid solution, arranged so as to be able to recover the liquid solution running off the receiving face (25) and communicating with a lower end (3) of the mount.

18. Biosensor according to one of claims 12 to 17, **characterised in that** it comprises means (3) for mounting the mount (2) on a receptacle for recovery of the liquid solution.

19. Electrochemical biosensor chip for measurement of the concentration of a compound in a liquid solution, **characterised in that** it comprises:
- a reagent chamber (8) enclosing a quantity of liquid biochemical composition, termed reactive composition, and having a semi-permeable membrane (9) closing the reagent chamber (8) so as to retain therein the reactive composition, this semi-permeable membrane (9) having a free outer face, termed receiving face (25), capable of receiving a drop of liquid solution separated from the reactive composition by the semi-permeable membrane (9),
- an electrode, termed first electrode (7), placed in electrical contact with the reactive composition contained in the reagent chamber (8), and means (17) for electrical connection of this first electrode (7) with an electrical circuit outside the chip (6).

20. Chip according to claim 19, **characterised in that** the first electrode (7) has an end (19) emerging in the reagent chamber (8) opposite a portion of the semi-permeable membrane (9) forming the said receiving face (25).

21. Chip according to one of claims 19 and 20, **characterised in that** the first electrode (7) forms a bottom (19) of the reagent chamber (8) which is closed, opposite this bottom (19), by the semi-permeable membrane (9).

22. Chip according to claim 21, **characterised in that** the reagent chamber (8) is delimited by the bottom (19) formed by the first electrode (7) and by the semi-permeable membrane (9) extending from the bottom (19) and above the bottom (19).

23. Chip according to one of claims 21 and 22, **characterised in that** it has a recessed groove (20) around the bottom (19) formed by the first electrode (7), this groove (20) being adapted to receive a peripheral seal (21) blocking the semi-permeable membrane (9) around and above this bottom (19).

24. Chip according to one of claims 19 to 23, **characterised in that** the first electrode (7) extends so as to have a portion (17) emerging outside the chip (6) in order to form means (17) for electrical connection with an external electrical circuit.

25. Chip according to one of claims 19 to 24, **characterised in that** it comprises a body (16) of electrically insulating synthetic material in the general shape of a small plate, and **in that** the first electrode (7) traverses the thickness of this body (16).

26. Chip according to one of claims 19 to 25, **characterised in that** it is in the general shape of a disc.

27. Chip according to one of claims 19 to 26, **characterised in that** it has a thickness of between 2 mm and 10 mm and a length dimension of between 5 mm and 50 mm.

28. Chip according to one of claims 19 to 27, **characterised in that** the first electrode (7) has a mean radial dimension of between 1 mm and 10 mm - in particular of the order of 4 mm.

29. Chip according to one of claims 19 to 28, **characterised in that** the reactive composition is an enzymatic aqueous solution.
